# EUROPEAN PATENT APPLICATION

(11) **EP 2 210 595 A1**
(43) Date of publication of application: **28.07.2010**
(21) Application number: 09150551.1
(22) Date of filing: 14.01.2009
(51) Int. Cl.: A61K 9/20, A61K 47/32

(54) **Active coating of pharmaceutical dosage forms**

(71) Applicant: LEK Pharmaceuticals d.d., 1526 Ljubljana (SI)
(72) Inventor: The designation of the inventor has not yet been filed
(74) Representative: Kosak, Alenka

(57) **Abstract**

The present invention describes in an embodiment a coating composition which contains in a film coating an active pharmaceutical ingredient, which is defined by a low water solubility of about 10mg/ml or lower as measured in water at 20°C at about pH 7, or which is defined by presenting a dispersed state when placed in water at 20°C at about pH 7, and a co-polymer of polyvinyl alcohol with polyethylene glycol. Preferably the active pharmaceutical ingredient has been applied dispersed in an aqueous coating vehicle onto a core which optionally comprises a same or different active pharmaceutical ingredient. The present invention also describes a process for the preparation of a pharmaceutical single unit dosage form, wherein the process comprised the steps of providing a core of the single unit dosage form, optionally providing one or more subcoating layer(s) on the core, subjecting the core to film coating using a composition comprising an aqueous coating vehicle, a co-polymer of polyvinyl alcohol with polyethylene glycol and at least one active pharmaceutical ingredient dispersed in the aqueous coating vehicle, wherein said co-polymer amounts for at least 7.0 wt.%, preferably at least 9.4 wt. % of said composition and the weight ratio of said co-polymer to said active pharmaceutical ingredient is at least 1:1 to 15:1. Other process embodiments are also described. High drug loads, uniformity of drug load, and fast dissolution rates of active pharmaceutical ingredient from film coatings of pharmaceutical single unit dosage forms can be achieved according to the present invention.

## Description

### Field of the Invention

Present invention relates to the field of pharmaceutical compositions, particularly to film coating comprising active pharmaceutical ingredient (sometimes abbreviated as API), i.e. a substance or a compound that is intended to be used for a pharmaceutical product as a therapeutically active compound (ingredient), and processes for the preparation of such pharmaceutical compositions or film coatings.

### Background of the Invention

In cases of incompatible active pharmaceutical ingredients (APIs), or for achieving a modified release profile, or just combining two or more different types of APIs for other purposes like better medicinal effect, it is desired to provide pharmaceutical composition with a film coating comprising active pharmaceutical ingredient. Incorporating active pharmaceutical ingredient in a film coating and separating it from the compounds in a core always presented a difficult task, especially as pharmaceutical compositions are subject to stringent scrutiny of pharmaceutical authorities and industry on matters such as residual organic solvents, stability, uniformity of API content, economical and environmental friendly manufacture. There is only limited knowledge about the feasibility of such matters in the art and essentially no marketed product that would comply with the above requirements.

Some attempts were made for film coating containing active pharmaceutical ingredients. US 6,656,503 B1 is about producing a pharmaceutical tablet comprising a core and a film coating, wherein the core comprises an nonsteroidal anti-inflammatory drug (NSAID) and the film coating comprises a polymer and misoprostol. Water is mentioned as a possible component of a coating vehicle, but only in a context that the resulting solvent system dissolves the misoprostol, and therefore the document specifically discloses the preferable use of a mixture of chlorinated hydrocarbon and an alcohol as the solvent system. Furthermore, preferred polymers selected according to US 6,656,503 B1 are povidone and water-soluble cellulose derivatives in a weight ratio from about 10 to about 100 parts polymer to 1 part misoprostol.

US 2004/0131791 A1 discloses a special process and apparatus for coating single or multiunit dosage forms. The necessity to use a special equipment is a disadvantage. Another disadvantage is that, as understood form the disclosure of 2004/0131791 A1, the coating comprises only low dose of an active substance.

WO 03/059327 and WO 2006/048208 each disclose bilayer tablets wherein each layer contains a different active pharmaceutical ingredient, namely telmisartan/hydrochlorothiazide according to WO 03/059327 and telmisartan/amlodipine according to WO 2006/048208. In both documents, a first tablet layer and a second tablet layer are prepared separately, and then compressed in a tablet press.

US5601843 discloses a pharmaceutical composition including a core containing an NSAID, which core is surrounded by a mantle coating of a prostaglandin. The disadvantage of mantle coating is that the final tablet is substantially larger than the inner core and the composition of the mantle resembles the composition of the tablet core as it must meet the flowability and compressibility requirements.

WO 2007/144175 discloses a pharmaceutical composition comprising a core with a first API I and at least one coating comprising a second API. It is evident from the specification that ethanol solutions of API were used for film coating. It further discloses cellulose derivatives, acrylic polymers, polyvinyl pyrrolidone and polyethylene glycol as possible polymers to be used for film coating.

US 2005/0013863 discloses film coated tablets comprising a first API in a core and a second API in a film coating. As polymers for use in forming the film, US 2005/0013863 mentions poly(ethylene oxide) hydroxypropyl methyl cellulose, poly(vinyl alcohol), combinations of poly(ethylene oxide) and hydroxypropyl methyl cellulose, and combinations of poly(vinyl alcohol) and poly(ethylene oxide). A co-polymer of polyvinyl alcohol with polyethylene glycol is also mentioned as a possible polymer to use. However, the API load in the coating is low; using glimepiride as API, in one coating the weight ratio of polymer to the API exceeds 18, in another coating it exceeds 37.
US 2008/020055A discloses a pharmaceutical composition comprising phenylephrine or a pharmaceutically acceptable salt thereof, i.e. a hydrophilic API, an erodible layer and optionally an outer coating which may again comprise phenylephrine. In an embodiment, the outer coating may contain polyvinyl alcohol-polyethylene glycol graft copolymer. The pharmaceutical composition is for oral administration wherein the composition delivers phenylephrine or a pharmaceutically acceptable salt thereof via absorption in the colon. The pharmaceutical composition can further comprise one or more additional therapeutically active agents selected from one or more of the group consisting of antihistamines, analgesics, anti-pyretics, and non-steroidal anti-inflammatory agents. An example of an antihistamine is loratadine.

Except the aforementioned US 2004/0131791 A1, which discloses a special process and apparatus for coating single unit dosage forms, none of the documents provide satisfactory data on uniformity of API content, which proves to be very difficult to achieve when employing film coating in a coating pan, especially when dispersed API is used.

The object of the present invention is to provide an improved pharmaceutical oral single unit dosage form, where API is present in a coating, and to provide an improved process for film coating of pharmaceutical compositions, such as pharmaceutical single unit dosage forms and in particular oral dosage forms such as tablets. Particularly, the object of the present invention is to provide stable, robust and safe pharmaceutical single unit dosage form comprising a core and a film coating.

### Summary of the Invention

In one aspect the present invention provides pharmaceutical single unit dosage form, comprising:
(i) a core, optionally comprising a first active pharmaceutical ingredient; and
(ii) a film coating comprising a second active pharmaceutical ingredient, which is defined by a low water solubility of about 10mg/ml or lower as measured in water at 20°C at about pH 7, or which is defined by presenting a dispersed state when placed in water at 20°C at about pH 7, and a co-polymer of polyvinyl alcohol with polyethylene glycol, wherein the weight ratio of said co-polymer to the second active pharmaceutical ingredient in the film coating lies in a range of 1:1 to 15:1.

In a further aspect the present invention provides a process for the preparation of a pharmaceutical single unit dosage form, the process comprising the steps of:
(aa) providing a core of the single unit dosage form;
(ba) optionally providing one or more subcoating layer(s) on the core; and
(ca) subjecting the core, optionally provided with one or more subcoating layer(s), to film coating using a composition comprising an aqueous coating vehicle, a co-polymer of polyvinyl alcohol with polyethylene glycol, and at least one active pharmaceutical ingredient dispersed in said aqueous coating vehicle, wherein said co-polymer amounts for at least 7.0 wt.%, preferably at least 9.4 wt. % of said composition and the weight ratio of said co-polymer to said active pharmaceutical ingredient lies in a range of 1:1 to 15:1.

In another aspect, the present invention further provides a process for preparation of a pharmaceutical single unit dosage form, the process comprising the steps of:
(ab) providing a core of the single unit dosage form in a coating pan,
(bb) optionally providing one or more subcoating layer(s) on the core, and
(cb) subjecting the core, optionally provided with one or more subcoating layer(s), to film coating using a composition comprising an aqueous coating vehicle, a co-polymer of polyvinyl alcohol with polyethylene glycol and at least one active pharmaceutical ingredient dispersed in said aqueous coating vehicle, wherein the weight ratio of said co-polymer to the active pharmaceutical ingredient lies in a range of 1:1 to 15:1, and wherein the coating pan rotates at 10 revolutions per minute or faster.

The present invention further provides a set of multiple pharmaceutical single unit dosage forms, wherein each dosage form unit of the set comprises a film coating comprising at least one active pharmaceutical ingredient and a co-polymer of polyvinyl alcohol with polyethylene glycol, wherein the weight ratio of the co-polymer to the active pharmaceutical ingredient in the film coating lies in a range of 1:1 to 15:1 and wherein the relative standard deviation of content variation of the active pharmaceutical ingredient in film coatings of said set is ≤ 7 %.

The present invention still further provides a use of a composition comprising a co-polymer of polyvinyl alcohol with polyethylene glycol and an aqueous coating vehicle, which comprises at least one active pharmaceutical ingredient, wherein said at least one active pharmaceutical ingredient is defined by a low water solubility of about 10mg/ml or lower as measured in water at 20°C at about pH 7, or which is defined by presenting a dispersed state when placed in water at 20°C at about pH 7, and the weight ratio of said co-polymer to the active pharmaceutical ingredient in the vehicle lies in a range of 1:1 to 15:1, in a film coating said active pharmaceutical ingredient onto a pharmaceutical single unit dosage form.

In the present invention, it was surprisingly found that a film coating comprising co-polymer of polyvinyl alcohol with polyethylene glycol significantly contributes to the stability of an API to be included into the film coating, even when the API itself has low solubility in water and despite the feasibility of a relatively high API load. This is especially critical in situations when only one or more lipophilic APIs are included in the film coating, especially when the second API is the only API included in the film coating. Further surprisingly, the resulting API-containing coating film, even when loaded at a high dose, shows a high uniformity of API content among different samples of the obtained dosage form. Unpredictably, the uniformity of API content was found to be enabled by using a weight ratio of the co-polymer of polyvinyl alcohol with polyethylene glycol and API in the film coating of only 15:1, and even at higher API loads defined by weight ratios up to 10:1, up to 5:1, up to 2:1, or even up to 1:1. A further relevant process-related factor that can be applied to reach superior uniformity of API content is using specific process parameters, wherein while observing commonly the presence of API in an aqueous coating vehicle containing the co-polymer of polyvinyl alcohol with polyethylene glycol, alternative technical measures shall be observed to commonly achieve improved uniformity of API: (i) a sufficient proportion of at least 7.0 wt.%, preferably at least 9.4 wt.-% of said co-polymer in said vehicle, or (ii) using a coating pan for film coating and applying a rotation speed of the coating pan of more than 10 revolutions per minute. This is even more surprising having regard to the finding that the API load uniformity can be achieved despite of the preferred possibility of using API in a dispersed state by using a coating vehicle which contains at least 25% w/w, preferably at least 40 % w/w, more preferably at least 50 % w/w, particularly at least 60 % w/w and more particularly at least 80 % w/w of water, or wherein said coating vehicle consists essentially or only of water. Dispersion state in water alternatively defines a proper selection of API to make use of the concept and effects of the present invention, similar to the critical situation when using API having low water solubility.

The combination of using a specific copolymer for film coating and the weight ratio of the copolymer to API was found to be especially advantageous. As a further surprising finding, it has been determined in the present invention that despite the lipophilic nature of the API that has low solubility in water in a film coating of a coated composition, a dissolution thereof is promoted when used in combination with co-polymer of polyvinyl alcohol with polyethylene glycol, even though the composition itself is less wetted due to lipophilicity of the thus selected API. It surprisingly appears that using co-polymer of polyvinyl alcohol with polyethylene glycol instead of more hydrophilic polymers like hydroxypropylmethyl cellulose or polyvinylpyrrolidone does accelerate the dissolution of the relatively lipophilic API. Co-polymer of polyvinyl alcohol with polyethylene glycol used for film coating, especially on water-based coating systems, leads to rapid disintegration of the film coating according to which dissolution rate of the coating film is beneficially fast, thereby enabling, for example, early start of release of API present in the core of the single dosage form.

As has been further found surprisingly in the course of using in an illustrative example hydrochlorothiazide as the lipophilic API having low water solubility for film coating, that a film coating comprising a co-polymer of polyvinyl alcohol with polyethylene glycol does not harm the stability of the API, even though the stability issue has been well known in cases where hydrochlorothiazide and polyethylene glycol are combined. The present invention is particularly valuable in the event that hydrochlorothiazide or its salt is selected as the API to be included into the film coating.

The present invention also resolves safety issues in cases where the composition comprises highly corrosive or generally reactive compounds, which would be otherwise exposed to the surface of the final pharmaceutical dosage form. This situation is known from the prior art, where a bilayer tablet comprises a certain first API requiring strongly basic substances such as sodium hydroxide for first API stabilization in one layer and another API in another layer. Such compositions containing basic stabilizing substances tend to be hygroscopic and takes up moisture while holding the bilayer tablet in hands, allowing the strongly basic substances such as sodium hydroxide to corrode skin while handling. Such defect can be easily overcome by depositing the film coating on the surface of the pharmaceutical dosage form representing a core, thereby protecting the end user from potentially reactive compounds in the core. As a consequence, the coating concept of the present invention may be applied when combining another API in the coating composition, but it can alternatively applied even in cases where the core is free of first API but is useful for the protection of core compositions to the outside, especially in cases as mentioned where the core composition contains any component selected from the group consisting of hygroscopic, strongly acid substances or strongly basic substances. "Strongly acid substances" used herein means the substances having pKa of below 5, preferably below 4. "Strongly basic substances" used herein means the substances having pKa of more than 9, preferably more than 10. "Hygroscopic" used herein means a characteristic for a substance when said substance absorbs at least 5 % of its weight water when exposed to 90 % relative humidity over 24 hours.
A typical situation where such problems may occur, and thus where the special coating concept according to the present can be applied, is when the core to be coated contains sartan-type APIs such as telmisartan.

Thus, the present invention provides improved processes for film coating cores of single unit dosage forms such as tablets, satisfying the need of convenient, fast, industrially applicable and reliable systems for the manufacture, yet at reasonably low costs, of pharmaceutical formulations having film coatings that contain at least one API. The present invention further provides improved single unit dosages forms which display particularly advantageous properties, such as safety, high API dose loading into the film coating, high content uniformity and fast or controlled dissolution rate, and this is surprisingly achieved even in critical cases wherein the API to be coated has characteristics of low solubility in water or is present in a dispersed state in water.

### Description of the invention, its advantages and preferred embodiments:

The aspects, advantageous features and preferred embodiments of the present invention summarized in the following items, respectively alone or in combination, further contribute to solving the object of the invention:
(1) A pharmaceutical single unit dosage form comprising
   (i) a core, optionally comprising a first active pharmaceutical ingredient, and
   (ii) a film coating comprising a second active pharmaceutical ingredient, which is defined by a low water solubility of about 10mg/ml or lower as measured in water at 20°C at about pH 7, or which is defined by presenting a dispersed state when placed in water at 20°C at about pH 7, and a co-polymer of polyvinyl alcohol with polyethylene glycol, wherein the weight ratio of the co-polymer to the second active pharmaceutical ingredient in the film coating lies in a range of 1:1 to 15:1.
(2) The pharmaceutical single unit dosage form according to (1), wherein said film coating is the outer coating.
(3) The pharmaceutical single unit dosage form according to (1) or (2), wherein said film coating comprises only, as said second pharmaceutically active ingredient, at least one active pharmaceutical ingredient which is defined by the low water solubility of about 10mg/ml or lower or which is defined by presenting a dispersed state when placed in water at 20°C at about pH 7, i.e. which is free of any hydrophilic active pharmaceutical ingredient being readily water-soluble.
(4) The pharmaceutical single unit dosage form according to any one of (1) to (3), wherein the weight ratio of the co-polymer to the second active pharmaceutical ingredient in the film coating lies in a range of 1:1 to 10:1, preferably 1:1 to 5:1, more preferably 1:1 to 2:1.
(5) The pharmaceutical single unit dosage form according to any one of the preceding items, wherein the film coating is deposited over the entire surface of the core.
(6) The pharmaceutical single unit dosage form according to any one of the precedings items, wherein the film coating is formed by using an aqueous coating vehicle in which the second active pharmaceutical ingredient is dispersed.
(7) The pharmaceutical single unit dosage form according to item (6), wherein the aqueous coating vehicle consists essentially of water, or consists of only water or of a mixture of organic solvent and at least 25% w/w, preferably at least 40 % w/w, more preferably at least 50 % w/w, particularly at least 60 %, more particularly at least 80 % w/w of water.
(8) The pharmaceutical single unit dosage form according to any one of the preceding items, wherein the film coating contains as excipient, besides said co-polymer and optionally further polymer(s), further only a stabilizer for said second active pharmaceutical ingredient.
(9) The pharmaceutical single unit dosage form according to any one of the preceding items, wherein the film coating further comprises citric acid.
(10) The pharmaceutical single unit dosage form according to any one of the preceding items, **characterized in that** the film coating comprises at least 1 mg, preferably at least 5 mg, more preferably at least 10 mg and even more preferably at least 20 mg of the second active pharmaceutical ingredient.
(11) The pharmaceutical single unit dosage form according to any one of the preceding items, characterized by exhibiting a dissolution profile according to which more than 20 %, particularly more than 40 %, yet more particularly more than 70 % of the second pharmaceutically active ingredient comprised in the film coating is released within 10 minutes, particularly within 5 minutes using USP apparatus 2, placing the pharmaceutical single unit dosage form in 900 mL of potassium phosphate buffer (pH=7.5) at 37°C with paddle speed of 75 rpm.
(12) The pharmaceutical single unit dosage form according to any one of the preceding items, wherein said first active pharmaceutical ingredient is same or different from said second active pharmaceutical ingredient.
(13) The pharmaceutical single unit dosage form according to any one of the preceding items, wherein said first and second active pharmaceutical ingredients are different and are incompatible.
(14) The pharmaceutical single unit dosage form according to any one of the preceding items, wherein said first active pharmaceutical ingredient is an angiotensin II antagonist, preferably selected from the groups consisting of sartans (preferably telmisartan, losartan, eprosartan, irbesartan, valsartan and candesartan, in particular telmisartan); cholesterol absorption inhibitors (preferably ezetimibe); and statins (preferably atorvastatin, simvastatin, rosuvastatin and lovastatin).
(15) The pharmaceutical single unit dosage form according to any one of the preceding items, wherein said second active pharmaceutical ingredient is a diuretic, preferably selected from the group consisting of hydrochlorothiazide, chlorothiazide and trichlorothiazide and their salts, particularly hydrochlorothiazide; or calcium channel blockers, preferably amlodipine, lercanidipine, nimodipine, nifedipine, verapamil and diltiazem.
(16) The pharmaceutical single unit dosage form according to any one of the preceding items, wherein the first active pharmaceutical ingredient is telmisartan and the second active pharmaceutical ingredient is hydrochlorothiazide; or the first active pharmaceutical ingredient is lovastatin and the second active pharmaceutical ingredient is niacin; or the first active pharmaceutical ingredient is simvastatin and the second active pharmaceutical ingredient is niacin.
(17) A pharmaceutical single unit dosage form, comprising:
   (i) a core comprising telmisartan as a first active pharmaceutical ingredient; and
   (ii) a film coating comprising hydrochlorothiazide as a second active pharmaceutical ingredient, and a co-polymer of polyvinyl alcohol with polyethylene glycol, wherein the weight ratio of said co-polymer to hydrochlorothiazide in the film coating lies in a range of 1:1 to 15:1.
(18) The pharmaceutical single unit dosage form according to any one of the preceding items, wherein the pharmaceutical single unit dosage form is for oral use.
(19) The pharmaceutical single unit dosage form according to any one of the preceding items, **characterized in that** the mass ratio of core to film coating is between 150:1 to 2.5:1.
(20) The pharmaceutical single unit dosage form according to any one of the preceding items, wherein one or more subcoating layer(s) is (are) provided between the core and the film coating.
(21) A process for the preparation of a pharmaceutical single unit dosage form, the process comprising the steps of:
   providing a core of the single unit dosage form;
   optionally providing one or more subcoating layer(s) on the core; and
   subjecting the core, optionally provided with one or more subcoating layer(s), to film coating using a composition comprising an aqueous coating vehicle, a co-polymer of polyvinyl alcohol with polyethylene glycol and at least one active pharmaceutical ingredient dispersed in said aqueous coating vehicle, wherein said co-polymer amounts for at least 7.0 wt.%, preferably at least 9.4 wt. % of said composition and the weight ratio of said co-polymer to the active pharmaceutical ingredient lies in a range of 1:1 to 15:1.
(22) A process for preparation of a pharmaceutical single unit dosage form, the process comprising the steps of:
   providing a core of the single unit dosage form in a coating pan;
   optionally providing one or more subcoating layer(s) on the core; and
   subjecting the core, optionally provided with one or more subcoating layer(s), to film coating using a composition comprising an aqueous coating vehicle, a co-polymer of polyvinyl alcohol with polyethylene glycol and at least one active pharmaceutical ingredient dispersed in said aqueous coating vehicle, wherein the weight ratio of said co-polymer to the active pharmaceutical ingredient lies in a range of 1:1 to 15:1 and the coating pan rotates at 10 revolutions per minute or faster.
(23) The process for the preparation of a pharmaceutical single unit dosage form according to item (22), wherein the coating pan rotates faster than 12 revolutions per minute, preferably faster than 15 revolutions per minute, more preferably faster than 17 revolutions per minute, even more preferably faster than 19 revolutions per minute.
(24) The process for the preparation of a pharmaceutical single unit dosage form according to any one of items (22) to (23), wherein the core and the film coating respectively contain the same or, preferably, different active pharmaceutical ingredient.
(25) The process for the preparation of a pharmaceutical single unit dosage form according to any one of items (22) to (24), wherein the core comprises an angiotensin II antagonist as a active pharmaceutical ingredient, preferably selected from the groups consisting of sartans such as telmisartan, losartan, eprosartan, irbesartan, valsartan and candesartan, more preferably telmisartan; cholesterol absorption inhibitors, preferably ezetimibe; and statins, preferably atorvastatin, simvastatin, rosuvastatin and lovastatin.
(26) The process for the preparation of a pharmaceutical single unit dosage form according to any one of items (22) to (25), wherein the film coating comprises a diuretic as a active pharmaceutical ingredient, preferably selected from the group consisting of hydrochlorothiazide, chlorothiazide and trichlorothiazide and their salts, more preferably hydrochlorothiazide; or calcium channel blockers, preferably amlodipine, lercanidipine, nimodipine, nifedipine, verapamil and diltiazem.
(27) The process according to any one of items (22) to (26), wherein the pharmaceutical single unit dosage form is for oral use.
(28) The process according to any one of items (22) to (27), wherein the aqueous coating vehicle consists essentially of water, or consists of only water or of a mixture of organic solvent and at least 25% w/w, preferably at least 40 % w/w, more preferably at least 50 % w/w, particularly at least 60 %, more particularly at least 80 % w/w of water.
(29) The process according to any one of items (22) to (28), **characterized in that** the mass ratio of core to film coating is in the range of from 150:1 to 2.5:1.
(30) The process according to any one of items (22) to (29) wherein the composition comprises at least 1 mg, preferably at least 5 mg, more preferably at least 10 mg and even more preferably at least 20 mg of said at least one active pharmaceutical ingredient.
(31) A set of multiple pharmaceutical single unit dosage forms, wherein each dosage form-unit of the set comprises a film coating comprising at least one active pharmaceutical ingredient and a co-polymer of polyvinyl alcohol with polyethylene glycol, wherein the weight ratio of the co-polymer to the active pharmaceutical ingredient in the film coating lies in a range of 1:1 to 15:1 and wherein the relative standard deviation of content variation of the active pharmaceutical ingredient in film coatings of said set is ≤ 7 %.
(32) The set of multiple pharmaceutical single unit dosage forms according to item (31), wherein each dosage form-unit of the set comprises a film coating obtained by providing a core of the single unit dosage form in a coating pan, optionally providing one or more subcoating layer(s) on the core, and subjecting the core, which is optionally provided with one or more subcoating layer(s), to film coating using a composition comprising an aqueous coating vehicle, a co-polymer of polyvinyl alcohol with polyethylene glycol and at least one active pharmaceutical ingredient dispersed in said aqueous coating vehicle, wherein the coating pan rotates faster than 15 revolutions per minute.
(33) The set of multiple pharmaceutical single unit dosage forms according to item (31) or (32), **characterized in that** the relative standard deviation of content variation of said active pharmaceutical ingredient in said set of multiple pharmaceutical single unit dosage forms is ≤ 6 %, more preferably ≤ 5 %.
(34) The set of multiple pharmaceutical single unit dosage forms according to any one of items (31) to (33), **characterized in that** the film coating of each dosage form-unit of the set comprises more than 1 mg, preferably more than 5 mg, more preferably more than 10 mg and even more preferably at least 20 mg of said at least one active pharmaceutical ingredient.
(35) The set of multiple pharmaceutical single unit dosage forms according to any one of items (31) to (34), wherein said at least one active pharmaceutical ingredient is a diuretic, preferably selected from the group consisting of hydrochlorothiazide, chlorothiazide and its salts, and trichlorothiazide, more preferably hydrochlorothiazide.
(36) Use of a composition comprising a co-polymer of polyvinyl alcohol with polyethylene glycol and an aqueous coating vehicle, which comprises at least one active pharmaceutical ingredient and the weight ratio of said co-polymer to the active pharmaceutical ingredient in the vehicle lies in a range of 1:1 to 15:1, in a process of film coating said active pharmaceutical ingredient onto a pharmaceutical single unit dosage form.
(37) Use of a composition comprising a co-polymer of polyvinyl alcohol with polyethylene glycol and an aqueous coating vehicle according item (36), wherein said at least one active pharmaceutical ingredient is defined by a low water solubility of about 10mg/ml or lower as measured in water at 20°C at about pH 7, or which is defined by presenting a dispersed state when placed in water at 20°C at about pH 7.
(38) Use of a composition comprising a co-polymer of polyvinyl alcohol with polyethylene glycol and an aqueous coating vehicle according to any one of items (36) to (37), wherein active pharmaceutical ingredient is a diuretic, preferably selected from the group consisting of hydrochlorothiazide, chlorothiazide and trichlorothiazide and their salts, more preferably hydrochlorothiazide.
(39) Use according to any one of items (36) to (37), wherein the film coating is spray coating.
(40) The use according to any one of items (36) to (38), wherein the pharmaceutical single unit dosage form is for oral use, preferably is designed as a tablet.
(41) Use according to any one of items (36) to (39), wherein the pharmaceutical single unit dosage form is a core comprising a active pharmaceutical ingredient, wherein the core and the film coating respectively contain the same or different active pharmaceutical ingredient.
(42) Use of a composition comprising a co-polymer of polyvinyl alcohol with polyethylene glycol and an aqueous coating vehicle, which comprises at least one active pharmaceutical ingredient and the weight ratio of said co-polymer to the active pharmaceutical ingredient in the vehicle lies in a range of 1:1 to 15:1, in an outer film coating said active pharmaceutical ingredient onto a pharmaceutical single unit dosage form.
(43) Use of a composition comprising a co-polymer of polyvinyl alcohol with polyethylene glycol and an aqueous coating vehicle according to item (42), wherein said at least one active pharmaceutical ingredient is defined by a low water solubility of about 10mg/ml or lower as measured in water at 20°C at about pH 7, or which is defined by presenting a dispersed state when placed in water at 20°C at about pH 7.
(44) Use according to any one of items (42) to (43), wherein said at least one active pharmaceutical ingredient is hydrochlorothiazide.

It is contemplated that every aspect of the present invention can be accomplished by using conventional equipment for film coating, for example using perforated pan or any equipment using spray or fluidized bed technique for coating, or the like. Spray coating of fluid and especially liquid coating compositions according to the present invention are particularly preferred. This is distinctly different from compression or mantle coating, e.g. of one tablet layer to another tablet layer, which can be advantageously avoided according to the present invention. Thus, the final size of the single unit dosage form obtained according the present invention can be reduced in comparison to corresponding mantle or compression coated single unit dosage forms.

The term "film coating" used herein means depositing API and one or more polymers in the process of coating a core of a dosage form, which is optionally a continuous process, wherein the API and optional one or more polymers are dissolved or dispersed in the coating vehicle while being deposited on a core. The composition used for film coating is typically in liquid state. If so, it is distinct from the composition used for mantle or compression coating, where substantially dry compositions are used. The difference in the state of each composition leads to necessity of unrelated excipients or substances for each process. For example, the composition for mantle or compression coating does normally require excipients that improve compressibility and provide for flow properties, but limit adhesiveness, whereas excipients providing only aforementioned properties are redundant in film coating. Instead, excipients like solubilizers or plasticizers, and liquid substances such as water or aqueous solvent mixtures may find their place in the process of film coating. The film coating contemplated according to the present invention, based on the desired dissolution characteristics to be achieved, normally constitutes the outer film coating of a pharmaceutical single unit dosage form.

The term "coating vehicle" used herein means a component of the film coating composition, which is different from the polymer and/or other excipient components or additives, and which is used to dissolve, not completely dissolve or disperse the active pharmaceutical ingredient. The polymer and/or other excipient components or additives, if used, may also be dissolved, not completely dissolved or dispersed in the coating vehicle.

The term "set of multiple pharmaceutical single unit dosage forms" used herein means a group of single unit dosage forms such as tablets, caplets or capsules collected from samples of the same production batch or lot. Variation of the content of the at least one active pharmaceutical ingredient among said set, in particular the relative standard deviation, may be calculated from 10 different samples such as 10 dosage forms (tablets/caplets/capsules) as being sufficiently representative for each set.

The term "low water solubility" used herein means solubilities in water (at 20°C at around pH 7) of about 10mg/ml or lower, preferably about 5 mg/ml or lower, and more preferably about 1.5 mg/ml or lower. Another definition of the characteristic of API to be favorably included in the coating film for the concept of the present invention is that the API does not completely dissolve, or is at least partially dispersed in the coating vehicle containing the aforementioned proportions of water or consisting essentially or totally of water. In order to display its desired effect on the dissolution of the relatively lipophilic second API, optionally on the uniformity of API content, the coating film according to the present invention may contain only the API defined by the low water solubility, or defined by the dispersed state in water. That is, the advantages of the present invention are particularly effective when the film coating may be free of any hydrophilic active pharmaceutical ingredient being readily water-soluble.

High dose or load of active pharmaceutical ingredient herein is understood as any dose of at least 1 mg, especially at least 5 mg, more specifically at least 10 mg and even more specifically at least 20 mg. Under uniformity of content is meant herein the relative standard deviation of plurality of 10 individually assayed active pharmaceutical ingredient contents of dosage form-coatings being less than 7 %, particularly less than 6 %, more particularly less than 5 %. The method for determining the relative standard deviation of an active pharmaceutical ingredient contents in general involves the complete dissolution of the film coating in a specific amount of media. Composition, pH and other conditions of the media are selected for each active ingredient and are apparent to the person skilled in the art to provide complete dissolution thereof. After the whole active pharmaceutical ingredient from the film coating is dissolved in the media, one measures the concentration in the said solution using equipment, which are known to person skilled in the art, such as HPLC or derivatives thereof, and calculates the starting content of active pharmaceutical ingredient in a film coating. Using measurements of 10 individual coated dosage forms one calculates the arithmetic mean, standard deviation and from it the relative standard deviation (RSD).

### Brief Description of the Drawings

Fig. 1 shows dissolution profiles of an API from tablet film coatings prepared according to examples 1, 2 and 3, illustrated by using hydrochlorothiazide as the API present in the outer film coating; and.
   Fig. 2 shows dissolution profiles of an API from tablet cores prepared according to examples 1, 2 and 3, illustrated by using telmisartan as the API present in the core.

### Detailed description of the Invention and Embodiments thereof

Separating an active pharmaceutical ingredient from the same or other active pharmaceutical ingredient or excipient in a final dosage form for reasons as incompatibility of compounds, looking for modified release of an active pharmaceutical ingredient, ensuring the beneficial effect of simultaneous application of compounds to a patient, and the like has always presented a challenge. Surprisingly we observed, that active pharmaceutical ingredient can be incorporated at high doses in the film coating of a pharmaceutical oral single unit dosage form, wherein the core comprises at least one active pharmaceutical ingredient and the film coating also comprises at least one active pharmaceutical ingredient, which is same or different from said active pharmaceutical ingredient in the core, and a co-polymer of polyvinyl alcohol with polyethylene glycol, wherein the weight ratio of the co-polymer and said active pharmaceutical ingredient in the film coating is at least 1 and up to 15. Moreover, a set of plural pharmaceutical single unit dosage forms such as plural tablets obtained in the process of the invention comply with a uniformity of content with respect to the coatings, by having a relative standard deviation of contents of the at least one active pharmaceutical ingredient in film coatings of different samples of the set of less than 7 %, particularly less than 6 %, more particularly less than 5 %, suitably measured with representative 10 assayed samples of the set.

In particular, it has been found that paying attention to the weight ratio of the co-polymer of polyvinyl alcohol with polyethylene glycol and the active pharmaceutical ingredient co-present in the film coating being in the range of 1: to 15:1, and even more in the range of 1:1 to 10:1, more preferably 1:1 to 5:1 and especially 1:1 to 2:1, significantly contributes to several advantages: The lower the ratio is, the thinner the coating film can be made. That this has been made feasible even in the case of relatively lipophilic API as for example illustrated by hydrochloritazide, is one of the surprising results of the present invention. At the same time, compared to a case where more polymer is present, dissolution of the API present in the core can start earlier. In addition, the process can be made overall shorter, making it more robust. All these effects further contribute to a uniform content of API.

Pharmaceutical oral single unit dosage forms are, among all pharmaceutical compositions, from the view of a patient and from the view of manufacturing the most preferred dosage form. They are used to administer the single or combination of active ingredients, and include tablets, caplets, capsules and the like. They can be in various forms. For example, they are bilayer or multiple layer tablets. Therefore, in the present invention the aforementioned pharmaceutical oral single unit dosage forms, as well as those being optionally further coated, or optionally modified in any way known to the person skilled in the art, serve as a core that is being film coated to obtain a pharmaceutical single unit dosage form comprising a core and a film coating according to the present invention.

The pharmaceutical formulation such as the tablet usually contains several ingredients, either in the core or in the coating film or both, the active pharmaceutical ingredient being the most important among them. Ingredients may be used to improve mechanic characteristic of the active pharmaceutical ingredient, such as bulk, improved flow, better compressibility, improved disintegration characteristics, flavoring, or enhanced appearance. Therefore, many types of excipients are used in tablet formulations to suffice for proper manufacture. They include binders, fillers, lubricants, disintegrants, surfactants, coloring agents and possible other excipients that improve flow, compression, hardness, taste, and tablet performance. This similarly applies to caplets. The manufacture of capsules on the other hand can comprise less excipients, as the active ingredient may only be blended with a filler and encapsulated in an outer capsule material such as gelatine, starch, or cellulosic material. The active principle composition can be encapsulated in the form of a liquid or solid. In case the capsules are filled with granules, pellets or even small tablets, similar techniques as to obtaining tablets apply, with additional encapsulation at the end.

The core of the pharmaceutical single unit dosage form according to the present invention such as tablets, capsules or the like can be prepared using standard procedures and equipment. Specifically, tablets can be obtained by direct compression when a group of ingredients can be blended, placed onto a tablet press, and made into a tablet without any of the ingredients having to be changed. Otherwise, dry or wet granulation is used instead. Dry granulation process comprises forming granules without using a liquid solution. The powders of active ingredient and excipients are compacted and densified. Dry granulation can be conducted on a tablet press using slugging tooling or on a roller compactor. The obtained briquettes or small pellets are further milled through a low-shear mill, finally blended and lead for a tablet compression. When powders alone do not compress, then they must be wet granulated. Wet granulation is the process of adding a liquid solution to powders. The liquid solution can comprise either water, organic solvents or solvent system comprising mixture of water and organic solvents. The excipients like binders can be added to liquid solution for wet granulation. The granules obtained are optionally dried, screened, sieved, and charged to the tablet press, which can be for example rotary tablet press. In addition, pellets can be prepared using dry or wet granulation techniques. Other possible way to prepare pellets is for example extrusion-spheronization method. Similarly pellets can be compressed in the tablet or encapsulated in the capsule.

According to the present invention, the excipient used for film coating is a co-polymer of polyvinyl alcohol with polyethylene glycol, wherein the weight ratio of the co-polymer to an active pharmaceutical ingredient used in the film coating is at least 1:1 up to 15:1. Said copolymer is especially required to obtain the film coating structure and achieve specific properties according to the present invention. Optionally additional excipients, which can be used for either the core or the film coating or both, of the pharmaceutical oral single unit dosage form will be apparent to the skilled person. Suitable excipients are, but are not limited to, starch (such as corn starch and pregelatinized starch), gelatin, sugars (such as sucrose, glucose, dextrose and lactose, powdered sugar), sugar alcohols (such as mannitol, sorbitol, xylol), polyethylene glycol, polyvinyl alcohol, povidone, waxes, natural and synthetic gums, polyvinylpyrrolidone, cellulosic polymers (such as hydroxypropyl cellulose, hydroxypropyl methyl cellulose, methyl cellulose, ethyl cellulose, hydroxyethyl cellulose, powdered cellulose, microcrystalline cellulose), organic salts and organic acids (such as magnesium stearate, calcium stearate, stearic acid, citric acid), inorganic salts and inorganic acids (such as calcium carbonate, calcium phosphate, calcium sulfate, sodium hydroxide), silicon dioxide (such as colloidal silicon dioxide, in particular Aerosil), titanium dioxide, talc and alumina, antioxidant agents, surfactants (including anionic, cationic, amphoteric, or nonionic), and pigments. Derivatives of the aforementioned excipients can also be used in the present invention. Citric acid is preferably selected as the additional excipient of the coating layer. Citric acid has been found to be beneficial in terms of stability exemplified with using hydrochloritazide as the API in the film coating.

In favor of advantages such as low total amount of excipients and thinness of the coating film, it is further preferred that the coating film contains only one effective type of excipient besides the polymer(s), more preferably only a stabilizer for the API present in the film coating, in particular citric acid.

According to a preferred embodiment, besides the at least one active pharmaceutical ingredient being included into the film coating, the same or a different active pharmaceutical ingredients is incorporated into the core such that the APIs in the coating and the core are separated in the pharmaceutical oral single unit dosage form. By this, a desired dissolution profile of one or different APIs can be achieved, incompatibility problems can be avoided, or the like. Desired dissolution profile is achieved for example if the film coat is rapidly disintegrated and rapidly releases active pharmaceutical ingredient, while the core is designed as a controlled release composition and releases active pharmaceutical ingredient in a controlled manner, e.g. sustained release matrix. For example, this is particularly useful for the treatment of diseases such as some infections or sleep disorders where the initial load of the active pharmaceutical ingredient is instantly or quickly released (notably from the coat), and the subsequently controlled release of the active pharmaceutical ingredient occurs (notably from the core). The present embodiment is also useful in a situation where one active pharmaceutical ingredient present in the coat needs to be released before the one present in the core. For example, this is useful when the active pharmaceutical ingredient in the core is subjected to P-glycoprotein efflux in the intestinal tract and the active pharmaceutical ingredient in the coat acts as an inhibitor of P-glycoprotein. Namely, P-glycoprotein decreases the bioavailability of active pharmaceutical ingredient present in the core, which can be minimized by the prior inhibition of P-glycoprotein with the active pharmaceutical ingredient released from the coat. The present invention also provides the solution for the situation where the active pharmaceutical ingredient from the core needs to be released almost simultaneously as the active pharmaceutical ingredient from the film coating. The present embodiment is able to provide for properties which enable to reduce the lag time for release of the active pharmaceutical ingredient from the core. Other possible conditions and variations to affect dissolution profile will be apparent to the person skilled in the art. The present invention thus preferably encompasses pharmaceutical oral single unit dosage form wherein two or more of the active pharmaceutical ingredients are incorporated within the pharmaceutical dosage form, at least one being in a core and at least one in a film coating.

In addition, when two or more active pharmaceutical ingredients are combined in single pharmaceutical oral single unit dosage form, possible interactions or incompatibilities among the active ingredients, and/or among the active ingredients and the excipients, may arise. Possible incompatibilities between different first and second active pharmaceutical ingredients herein may encompass any chemical or physical change of any of the active pharmaceutical ingredients. Such interactions or incompatibilities may be avoided by separating active ingredients from one another, e.g. introducing one in the core and the other in the film coating. The core and the coat containing active pharmaceutically ingredient may be separated by an intermediate coat. The incompatibility or interactions of active pharmaceutical ingredients and the need for separating them will be apparent to those skilled in the art. For example considering a particular API combination of telmisartan, an antihypertensive compound, and hydrochlorothiazide, a diuretic, it is to be noted that telmisartan is normally blended with basic excipient like sodium hydroxide and meglumine in order to promote its dissolution, whereas hydrochlorothiazide is incompatible with environment of high pH, namely high pH environment leads to the degradation of hydrochlorothiazide and should therefore be protected therefrom.

In general, in order to asses the incompatibility issue, one can perform stability test using binary mixtures of two active principles or of an active principle and an excipient in a ratio from 1:1 to any ratio intended to be later in the final dosage form. The mixture is left for a period of 1 day, 1 week, 1 month, 6 month or 1 year under normal or stress conditions, wherein normal conditions mean room temperature (22 °C) and relative humidity of up to 65 %, while stress conditions mean heating to 40 °C or 60 °C and relative humidity of over 65 %, particularly of 85 %, more particularly of 95 %. After the aforementioned period passed, one can inspect the mixture using analytical methods known in the art, such as for example HPLC or X-ray, and evaluate the result of incompatibility, namely the degradation products, drop in concentration, change of color, smell, crystalline structure, or the like. The present invention thus particularly encompasses pharmaceutical oral single unit dosage form wherein two or more of the active pharmaceutical ingredients are separated from each other within the pharmaceutical dosage form by incorporating incompatible compounds separately in a core and a film coating.

In the present invention, a film coating for a pharmaceutical oral single unit dosage form or core is provided, wherein film coating comprises at least one active pharmaceutical ingredient. The polymer used in the film coating is a co-polymer of polyvinyl alcohol with polyethylene glycol, wherein the weight ratio of the co-polymer to an active pharmaceutical ingredient used in the film coating is at least 1:1 and up to 15:1. In addition, any natural or synthetic polymer that has proven to be suitable coating material may be used, providing it complies with the active pharmaceutical ingredient stability and desired release behavior, when sprayed onto a core. A suitable polymer includes povidone, polyvinyl pyrrolidone, polyvinyl alcohol, polyethylene glycol, cellulose, poly(meth)acrylic acid, their derivatives and co-polymers, as well as mixtures thereof, without being limited thereto. The additional polymer is preferably selected from the group consisting of hydroxypropyl methylcellulose, hydroxypropyl cellulose, hydroxyethyl cellulose, methylcellulose, carboxymethyl ethylcellulose, hydroxypropyl methylcellulose acetate succinate, polyvinyl alcohol, esters thereof like acetate esters such as polyvinyl acetate, and co-polymers thereof. The additional polymer may especially be selected to be highly water soluble as a favorable combination with the co-polymer of polyvinyl alcohol with polyethylene glycol, such as those types of polyvinyl alcohol polymer, poly(meth)acrylic acid, cellulose or polyethylene glycol respectively having high water solubility characteristics. Particularly preferred polymers may be selected from the group consisting of polyvinyl alcohol, *N*-(2-hydroxypropyl)methacrylamide (HPMA), and derivatives or copolymers thereof.

The coating vehicle suitable to disperse the active pharmaceutical ingredient and/or the polymer contains at least 25 %, particularly at least 40 %, more particularly at least 50 %, yet more particularly at least 60 % and even at least 80 % of water relative to the whole coating vehicle, wherein the percentage means w/w. Preferably the coating vehicle is a mixture of water and one or more organic solvent. Essentially only water or entirely only water can be used as coating vehicle to disperse the active pharmaceutical ingredient and/or the polymer. When used, the most suitable organic solvent used beside water is alcohol, particularly ethanol.

The present invention is suitable for practically any active pharmaceutical ingredient (API), as the film coating comprising the same is manageable independently even if dispersed API compounds in the coating vehicle is used in film coating according to the present invention. The choice of a co-polymer of polyvinyl alcohol with polyethylene glycol, wherein the weight ratio of the co-polymer to an active pharmaceutical ingredient used in the film coating is at least 1:1 and up to 15:1 allows the coating of practically all active pharmaceutical ingredient. Having the possibility and preferably making use of dispersing the active ingredient in the coating vehicle leads to loading higher amounts of active pharmaceutical ingredient into the film coating. The content of active pharmaceutical ingredient in a film coating is no longer limited to lower API load or event sub-milligram doses, which is a problem of conventional film coatings. Rather, doses of over 1 milligrams (mg), preferably over 5 mg and even over 10 mg can be easily incorporated into the film coating per single unit dosage form, e.g. per single tablet. In order to obtain doses of over 1 mg per single unit dosage form, the active pharmaceutical ingredient is dispersed in the film coating composition used according to the present invention. Therefore, achieving a higher content of the active ingredient is feasible by the process disclosed herein. The obtained film coating is also provided according to the present invention. The use of a co-polymer of polyvinyl alcohol with polyethylene glycol, wherein the weight ratio of the co-polymer and an active pharmaceutical ingredient used in the film coating is at least 1 and up to 15, in combination with at least 25 % of w/w, particularly at least 40 %, more particularly at least 50 %, yet more particularly at least 60 %, most preferably at least 80 % of water as a coating vehicle, or the use of a coating vehicle consisting of only water besides the preferred use of the coating polymer corresponds to stringent and valuable economical, environmental and pharmaceutical needs. The use of organic solvents can be minimized thereby.

A particularly preferred embodiment of the present invention and very advantageous feature over any prior art dual API systems is when telmisartan is used in the core of the pharmaceutical single dosage form and hydrochlorothiazide is used as an active pharmaceutical ingredient in the film coating. Doses of over 1 mg, preferably over 5 mg, more preferably over 10 mg and even over 20 mg of hydrochlorothiazide can be incorporated in a film coating, wherein water even without organic solvents as a coating vehicle is used and hydrochlorothiazide is dispersed in the said before spraying it onto the core. The ratio of a co-polymer of polyvinyl alcohol with polyethylene glycol to hydrochlorothiazide by weight may preferably be adjusted in a range from at least 1 and up to 15, preferably up to 10, particularly from at least 1 to up to 2. The ratio depends on the drug load that is desired to be achieved. In cases when film coating is intended to comprise for example just over 1 mg (less than 5 mg) of active pharmaceutical ingredient, the ratio is higher, but not limited to over 10, whereas for incorporation of doses over 5 mg, the ratio is lower, but not limited to under 10. The ratio depends also on the coating vehicle used, optionally added plasticizer, and desired release profile. Plasticizer may be for example polyol (such as glycerol, macrogol, propylene glycol), organic ester (such as citrate ester, phthalate ester, dibutyl sebacetate), oil/glyceride (such as castor oil, acetylated monoglycerides), or a combination thereof. Optionally, colourants, pigments, opacifiers, flavours, surfactants, acidifying agents (such as citric acid) and waxes can be added. Different formulations and processing parameters can be varied, such as coating vehicle, type of polymer and type and amount of added plasticizer or other mentioned excipients. The effect of different formulation and parameters in accordance to active pharmaceutical ingredient, its dose and desired release profile are well within the purview of those skilled in the art of pharmaceutical formulation. In addition, according to the present invention, it is possible to deliver all special characteristics of film coating, such as for example acido-resistance, the outlook of the formulation, protection of formulation from light or oxygen, protection of the patient from the active principle in a composition, simultaneously to spraying active ingredient onto the core in a single step, which is most advantageous over for example in the case of mantle or compression tablet, where additional steps for achieving the said characteristics are needed. It is evident from the present disclosure, that the film coating according to the present invention can be additionally coated, or be coated on a subcoat, or be pilled on another film coating or a different coated base comprising pharmaceutical ingredient.

Although other dual API combinations can be contemplated by those skilled in the art while paying attention to the conditions and advantages disclosed herein, further preferred embodiments include when lovastatin is used in the core and niacin is used in the film coating; or when simvastatin is used in the core and niacin is used in the film coating.

The loading of more than 1 mg of active ingredient in the film coating per single unit dosage form is a demanding task, if one uses dispersed active pharmaceutical ingredient in the film coating, being dispersed as a consequence from using at least 25 % of w/w, preferably at least 40 %, more preferably at least 50 %, particularly at least 60 %, yet more particularly at least 80 % w/w of water and even most preferably consists essentially or entirely only of water as a coating vehicle, since higher mass load of active ingredient normally means prolongation of the process of coating, while water renders film coating difficult to dry. Improper continuous drying of the product during the process gives unevenly coated cores, which returns final dosage form without uniformity of content. The situation is not as troublesome when organic solvents are used. Therefore, when at least 25 % of w/w, preferably at least 40 %, more preferably at least 50 %, yet more preferably at least 60 %, particularly at least 80 % w/w of water and even most preferably consists essentially or entirely only of water as a coating vehicle is used, a co-polymer of polyvinyl alcohol with polyethylene glycol, wherein the weight ratio of the co-polymer and an active pharmaceutical ingredient used in the film coating is at least 1 and up to 15, preferably at least 1 and up to 10, more preferably at least 1 and up to 2, in concordance with the used API compound load and the proportion of water in the coating composition should be selected.

The product of present invention can satisfy beneficial uniformity of content criteria. The relative standard deviation of contents of at least one active pharmaceutical ingredient in the coating film of a plurality of 10 dosage form-coatings can be controlled to less than 7 %, particularly less than 6 %, more particularly less than 5 %. To satisfy the criteria of uniformity of content, a co-polymer of polyvinyl alcohol with polyethylene glycol is used in the process of coating as coating vehicle, wherein the weight ratio of the co-polymer to an active pharmaceutical ingredient used in the film coating is at least 1:1 up to 15:1, preferably at least 1:1 up to 10:1, more preferably at least 1:1 up to 5:1, and particularly at least 1:1 and up to 2:1.,Furthermore, the dose of active pharmaceutical ingredient can be set to more than 1 mg, preferably more than 5 mg, more preferably more than 10 mg and even more preferably more than 20 mg. The process and products of the present invention disclosed herein is highly advantageous over prior art attempts to obtain film coatings in terms of comprising more than 1 mg of at least one active pharmaceutical ingredient, satisfying beneficial uniformity of content criteria, and in addition enabling a mass ratio of core to film coating to lie in the range of 150:1 to 2.5:1. A process for the preparation of a pharmaceutical single unit dosage form that gives best results, is the process comprising the steps of providing a core of the single unit dosage form, and subjecting the core to film coating using a composition comprising an aqueous coating vehicle, a co-polymer of polyvinyl alcohol with polyethylene glycol and dispersed at least one active pharmaceutical ingredient, wherein said co-polymer amounts for at least 7.0 wt.%, preferably at least 9.4 wt. % of said composition and the weight ratio of said co-polymer to said active pharmaceutical ingredient is at least 1:1 up to 15:1. Another possible variation, or a condition set in combination of the process applied to achieve the result is to provide a core of the single unit dosage form in a coating pan, and subject the core to film coating using a composition comprising an aqueous coating vehicle, a co-polymer of polyvinyl alcohol with polyethylene glycol and at least one active pharmaceutical ingredient dispersed in the coating vehicle, wherein the weight ratio of said co-polymer to the active pharmaceutical ingredient is at least 1:1 up to 15:1 and the coating pan rotates at 10 revolutions per minute or faster. The rotation velocity was found to contribute to end uniformity of the API content. The rotation 10 revolutions per minute or faster, and preferably of more than 12 revolutions per minute is advantageous, and more than 17 being preferred, more than 19 being even more preferred. The rotation speed may also depend on the volume of the coating drum. Specifically, when the coating device has a coating drum volume of 4.9 L or higher, for example when using an O'Hara lab coat or another lab coat, the rotation speed may be higher such as at least 13, more preferably more than 17 or even more than 19 revolutions per minute. When on the other hand the coating device has a coating drum volume of higher magnitude such as 30 L or higher or even 50 L or higher, for example when using Glatt GC 750, the rotation speed may be adjusted in a range of 10 to 13 revolutions per minute. Generally, the rotation speed may be adjusted lower with the tendency of a higher coating drum volume in order to still achieve a desirable uniformity of API content.

Aforementioned aqueous coating vehicle as used in the process of film coating is the vehicle wherein at least 25 % of w/w, preferably at least 40 %, more preferably at least 50 %, yet more preferably at least 60 %, particularly at least 80 % w/w of water and even most preferably consists essentially or entirely only of water. The process according to the present invention provides film coated pharmaceutical single unit dosage forms that are comparably dry to the known prior art dosage forms, even though aqueous vehicle of essentially or entirely only of water is used. For example, the content of water of bilayer tablet Micardis, which contains telmisartan in one tablet layer and hydrochlorothiazide in another, and of film coated tablets according to the present invention was measured by loss-on-drying methodology. The results for both showed up to about 2.7 % of water and did not differ significantly.

Another advantage of the present invention is immediate dissolution of the API compound from the film coating which comprises more than 1 mg of at least one active pharmaceutical ingredient. The film coating can exhibit a dissolution profile according to which more than 20 %, particularly more than 30 %, more particularly more than 40 %, yet more particularly more than 70 % of active pharmaceutical ingredient is released within 10 minutes, particularly within 5 minutes, when determined by reference using USP apparatus 2, placing the tablet in 900 mL of potassium phosphate buffer (pH=7.5) at 37°C with paddle speed of 75 rpm. The dissolution profile shows up to 85 % release of the active pharmaceutical ingredient in first 5 minutes using the aforementioned testing method. It is understood that the media of determining the dissolution profile can be changed according the active pharmaceutical ingredient, as the said should be soluble in the dissolution media. The advantage of fast dissolution rate of the coating is especially important, as the dissolution of the core can usually only proceed after dissolving the film coating. The preferred dissolution profile could be obtained even when rather lipophilic API is used in the film coating, illustrated by the preferred API hydrochlorothiazide.

A specific embodiment of the present invention is a film coated tablet, which comprises telmisartan in the core and hydrochlorothiazide in the coating. Coating is performed using a co-polymer of polyvinyl alcohol with polyethylene glycol, wherein the weight ratio of the co-polymer to hydrochlorothiazide in the film coating is at least 1:1 up to 15:1. In this preferred embodiment, hydrochlorothiazide is dispersed in a coating vehicle comprising at least 60 % of water, the rest being ethanol, or essentially only water or entirely only water, together with the polymer and sprayed on the tablet cores.

Generally, the present invention can be employed in manufacture using standard coating procedures and equipment. Such procedures are known to those skilled in the art and can be applied using a perforated coating pan, spray or fluidized coating equipment, or the like.

The following examples are merely illustrative of the present invention and they should not be considered as limiting the scope of the invention in any way, as these examples, modifications and other equivalents thereof will become apparent to those versed in the art in the light of the present disclosure, and the accompanying claims.

### Example 1

Composition of core

| | | |
|---|---|---|
| Telmisartan | 80.00 | mg |
| Sodium Hydrodroxide | 6.720 | mg |
| Meglumine | 24.00 | mg |
| Povidone K25 | 48.00 | mg |
| Granules of lactose monohydrate, povidone 30 and crospovidone (Ludipress) | 80.00 | mg |
| Lactose, Anhydrous | 237.28 | mg |
| Magnesium stearate | 4.00 | mg |
| Tablet mass: | 480.00 | mg |

### Preparation of core

The process of production of tablet core included dissolving telmisartan in 1 N NaOH and adding meglumine. Ethanol and povidone were further added to solution and povidone was gradually dissolved. Prepared solution was applied on Ludipress by spraying in fluid bed chamber. The obtained granulate was dried and screened through sieve. Additionally, granulate was homogenized with anhydrous lactose. Mg-stearat was added to the blend and mixed for a short time. Finally, tablets were compressed.

### Preparation of film coated tablets

| Subcoating | |
|---|---|
| Polyvinylpyrrolidone | 10.00 mg |
| | 10.00 mg |

| Coating | |
|---|---|
| polyvinyl alcohol-polyethylene | |
| glycol copolymer (Kollicoat IR) | 30.00 mg |
| Citric Acid | 1.00 mg |
| Hydrochlorothiazide | 12.50 mg |
| | 43.50 mg |

A batch of film coated tablets was prepared according to following procedure:

Dispersion for subcoating was made by dissolving (10 min.) polyvinylpyrrolidone (110.00 g; K25, Basf) in mixture of purified water (66.00 g) and ethanol (220.00 g). Dispersion for coating with active ingredient was prepared as follows:
in first beaker 330.00 g of Kollicoat IR (white; Basf) was dispersed in the 871.00 g water at room temperature, the room temperature being understood as 22°C, while mixing for 35 minutes. In another beaker 11.00 g of citric acid was dissolved in 22.00 g of purified water by mixing for 5 minutes. In a third beaker 137.50 g of hydrochlorothiazide was dispersed and
homogenized in 206.30 g of purified water using homogenizer (Ultraturax) for 30 minutes. After that mixtures of all three beakers were mixed together and stirred for more than 20 minutes.

First, the dispersion for subcoating was sprayed onto cores in a perforated pan so that the film coating in a weight ratio of about 2.0 w/w % in regard to coated core was obtained.
In the next step the dispersion comprising the active ingredient was sprayed onto cores comprising the first coating in the same perforated pan so that the film coating in a weight ratio of about 8.2 w/w % in regard final mass was obtained. During the coating process the tablet weight and the loss on drying of film coated tablets were controlled, meaning that the gain in weight was always calculated on the dried cores and dried coated cores, respectively.

### Comparative example 2

The composition of the cores and the method of their preparation was the same as presented in Example 1.

Preparation of film coated tablets

| Hydroxypropylmethylcellulose (HPMC) coating | |
|---|---|
| Hydrochlorothiazide | 12.50 mg |
| Pharmacoat 606 | 18.00 mg |
| Klucel EF | 9.00 mg |
| PEG 400 | 6.00 mg |
| Pigment | 0.50 mg |
| | 46.00 mg |

All components were dispersed in solution of purified water (80 mg per tablet) and ethanol (240 mg per tablet). Resulting dispersion was sprayed on tablet cores in perforated pan till weight of exemplary tablets gained 46 mg per tablet determined after drying. Coated tablets were subsequently dried in a pan.

### Comparative example 3

The composition of the cores and the method of their preparation was the same as presented in Example 1.

Preparation of film coated tablets

| Polyvinylpyrrolidone (PVP) coating | |
|---|---|
| Hydrochlorothiazide | 12.50 mg |
| Aerosil 200 | 12.50 mg |
| Citric Acid, anhydrous 1.26 mg | |
| Povidone (K25) | 43.70 mg |
| Pigment | 0.04 mg |
| | 70.0 mg |

All components were dispersed in solution of demi water (57mg per tablet) and Ethanol (130 mg per tablet). Resulting dispersion was sprayed on tablet cores in perforated pan till weight gain of 70 mg per tablet determined after drying. Coated tablets were subsequently dried in pan.

### Example 4

The uniformity of content of hydrochlorothiazide (HCTZ) in film coatings of different tablets of the preparation was determined in coated tablets prepared as described under examples 1 and 2. 10 coated tablets were randomly selected and the content of hydrochlorothiazide measured in their coatings. Average, standard deviation and relative standard deviation of the plurality of 10 contents were calculated.

| | **Coated tablets from example 1** | | **Coated tablets from example 2** | |
|---|---|---|---|---|
| | **Content of HCTZ (mg)** | **Percentage of theoretical content** | **Content of HCTZ (mg)** | **Percentage of theoretical content** |
| 1. | 12.25 | 98.00 | 12.13 | 97.00 |
| 2. | 12.21 | 97.70 | 11.21 | 89.70 |
| 3. | 13.35 | 106.80 | 11.48 | 91.80 |
| 4. | 12.18 | 97.40 | 11.75 | 94.00 |
| 5. | 12.76 | 102.10 | 11.34 | 90.70 |
| 6. | 12.79 | 102.30 | 10.31 | 82.50 |
| 7. | 13.25 | 106.00 | 10.75 | 86.00 |
| 8. | 13.31 | 106.50 | 10.43 | 83.40 |
| 9. | 12.78 | 102.20 | 11.61 | 92.90 |
| 10. | 11.56 | 92.50 | 9.51 | 76.10 |
| Arithmetic mean | 12.64 | 101.15 | 11.05 | 88.41 |
| Standard deviation | 0.59 | 4.69 | 0.79 | 6.33 |
| RSD* (%) | 0.046 | 4.6 | 0.072 | 7.2 |

| | | | | |
|---|---|---|---|---|
| *RSD - Relative standard deviation | | | | |

The results clearly show that uniformity of content can be achieved when following the process requirements of the present invention. Using hydroxypropylmethylcellulose as coating polymer produced the coatings with HCTZ contents with 7.2 % of relative standard deviation. Changing to co-polymer of polyvinyl alcohol with polyethylene glycol improved the result and obtained coatings with HCTZ contents showing superior relative standard deviation of only 4.6 %.

### Example 5

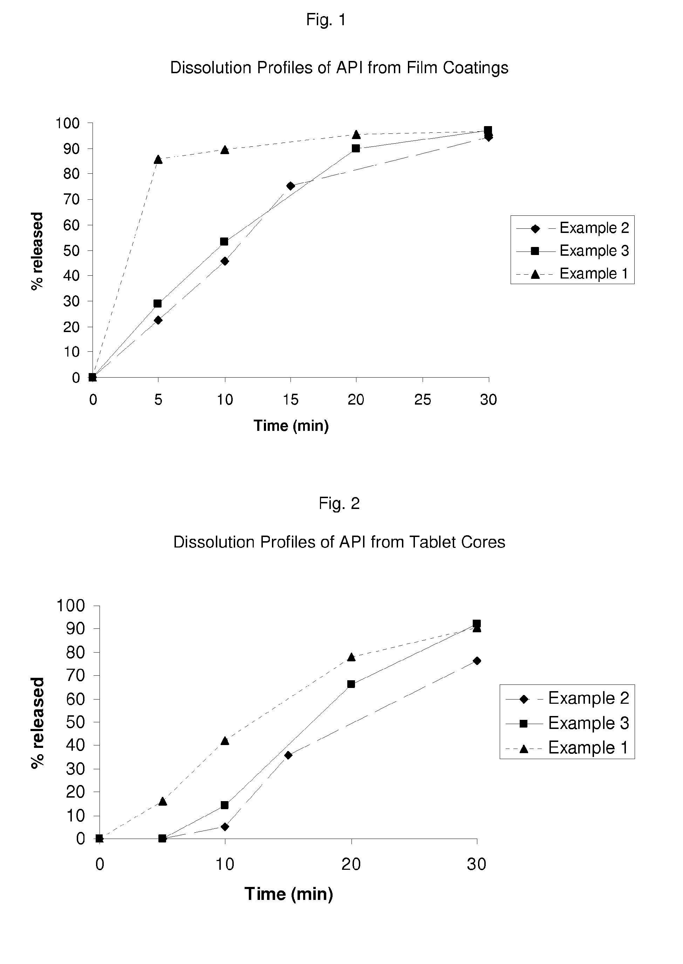

Film coated tablets prepared according the examples 1, 2 and 3 were charged to USP apparatus 2, placing the tablets in 900 mL of potassium phosphate buffer (pH=7.5) at 37°C with paddle speed of 75 rpm., and their dissolution profiles were determined. Fig. 1 and Fig. 2 show the dissolution profile of HCTZ dissolution from the film coating and telmisartan dissolution from the core, respectively.

As shown in Fig. 1 the release of hydrochlorothiazide from coated tablets prepared with a co-polymer of polyvinyl alcohol with polyethylene glycol (Example 1) is significantly faster compared to the coated tablets prepared with hydroxypropylmethylcellulose (Example 2) or with polyvinylpyrrolidone (Example 3). As a result of a faster dissolution of the tablet film coating, the release of telmisartan from the tablet core of the coated tablets is also faster. These results clearly show that besides the more robust technology, when using a co-polymer of polyvinyl alcohol with polyethylene glycol compared to hydroxypropylmethylcellulose and polyvinylpyrrolidone, a co-polymer of polyvinyl alcohol with polyethylene glycol also allows much faster release of the drugs from the coated tablets.

### Examples 6 to 8

The composition of the cores and the method of their preparation was the same as presented in Example 1. Also, the process for preparing the film coating was repeated to obtain the composition shown below. Relative standard deviation of 10 contents according to example 4 were calculated for compositions of examples 6 to 8.

| | **Example 6** | **Example 7** | **Example 8** |
|---|---|---|---|
| **Subcoating** | | | |
| polyvinyl alcohol-polyethylene glycol copolymer | 6 mg | 6 mg | 6 mg |

| **Coating** | | | |
|---|---|---|---|
| polyvinyl alcohol-polyethylene glycol copolymer | 25.0 | 25.0 | 25.0 |
| Hydrochlorothiazide | 12.5 | 25.0 | 12.5 |
| Citric Acid | 2.0 | 2.0 | 2.0 |
| **Coating pan rotation velocity** | 17 rpm* | 19 rpm* | 20 rpm* |
| **RSD**** | 6.3 | 4.5 | 4.3 |

| | | | |
|---|---|---|---|
| * rpm - Revolutions per minute ** RSD - Relative standard deviation | | | |

Example 6 to 8 show that using a co-polymer of polyvinyl alcohol with polyethylene glycol, wherein the weight ratio of the co-polymer and active pharmaceutical ingredient in the film coating is most preferably at least 1 and up to 2, yields pharmaceutical final dosage forms with uniformity of active pharmaceutical content of less than 7. The uniformity of active pharmaceutical ingredient content can be further influenced to drop by increasing the speed of coating pan rotation. Using the speed of 17 rpm provided for the uniformity of active pharmaceutical content expressed in relative standard deviation of 6.3, whereas increasing the rotation speed of coating pan to 19 or even 20 rpm, provided the relative standard deviation of 4.5 and 4.3, respectively.

## Claims

1. A pharmaceutical single unit dosage form, comprising:
(i) a core, optionally comprising a first active pharmaceutical ingredient; and
(ii) a film coating comprising a second active pharmaceutical ingredient, which is defined by a low water solubility of about 10mg/ml or lower as measured in water at 20°C at about pH 7, or which is defined by presenting a dispersed state when placed in water at 20°C at about pH 7, and a co-polymer of polyvinyl alcohol with polyethylene glycol, wherein the weight ratio of said co-polymer to the second active pharmaceutical ingredient in the film coating lies in a range of 1:1 to 15:1.

2. The pharmaceutical single unit dosage form according to claim 1, wherein the weight ratio of said co-polymer to said second active pharmaceutical ingredient in the film coating lies in a range of 1:1 to 10:1, preferably 1:1 to 5:1 and particularly 1:1 to 2:1.

3. The pharmaceutical single unit dosage form according to claim 1 or 2, wherein the film coating is formed by using an aqueous coating vehicle comprising dispersed second active pharmaceutical ingredient, wherein the aqueous coating vehicle comprises essentially water or only water or a mixture of organic solvent and at least 25% w/w, preferably at least 40 % w/w, more preferably at least 50 % w/w, particularly at least 60 %, more particularly at least 80 % w/w of water.

4. The pharmaceutical single unit dosage form according to any one of claims 1 to 3, wherein the film coating further comprises citric acid.

5. The pharmaceutical single unit dosage form according to any one of the preceding claims, **characterized by** exhibiting a dissolution profile according to which more than 20 %, particularly more than 40 %, yet more particularly more than 70 % of the second pharmaceutically active ingredient comprised in the film coating is released within 10 minutes, particularly within 5 minutes using USP apparatus 2, placing the pharmaceutical single unit dosage form in 900 mL of potassium phosphate buffer (pH=7.5) at 37°C with paddle speed of 75 rpm..

6. The pharmaceutical single unit dosage form according to any one of the preceding claims, wherein said first active pharmaceutical ingredient is contained and is an angiotensin II antagonist, preferably selected from the groups consisting of sartans (preferably telmisartan, losartan, eprosartan, irbesartan, valsartan and candesartan), cholesterol absorption inhibitors (preferably ezetimibe); and statins (preferably atorvastatin, simvastatin, rosuvastatin and lovastatin), most preferably being telmisartan.

7. The pharmaceutical single unit dosage form according to any one of the preceding claims, wherein said second active pharmaceutical ingredient is a diuretic, preferably selected from the groups consisting of hydrochlorothiazide, chlorothiazide and trichlorothiazide and their salts, more preferably hydrochlorothiazide; or calcium channel blockers, preferably amlodipine, lercanidipine, nimodipine, nifedipine, verapamil and diltiazem; in particular wherein said second active pharmaceutical ingredient is hydrochlorothiazide or its salt.

8. A process for the preparation of a pharmaceutical single unit dosage form, the process comprising the steps of:
(aa) providing a core of the single unit dosage form;
(ba) optionally providing one or more subcoating layer(s) on the core; and
(ca) subjecting the core, optionally provided with one or more subcoating layer(s), to film coating using a composition comprising an aqueous coating vehicle, a co-polymer of polyvinyl alcohol with polyethylene glycol, and at least one active pharmaceutical ingredient dispersed in said aqueous coating vehicle, wherein said co-polymer amounts for at least 7.0 wt.%, preferably at least 9.4 wt. % of said composition and the weight ratio of said co-polymer to said active pharmaceutical ingredient lies in a range of 1:1 to 15:1.

9. A process for preparation of a pharmaceutical single unit dosage form, the process comprising the steps of:
(ab) providing a core of the single unit dosage form in a coating pan,
(bb) optionally providing one or more subcoating layer(s) on the core, and
(cb) subjecting the core, optionally provided with one or more subcoating layer(s), to film coating using a composition comprising an aqueous coating vehicle, a co-polymer of polyvinyl alcohol with polyethylene glycol and at least one active pharmaceutical ingredient dispersed in said aqueous coating vehicle, wherein the weight ratio of said co-polymer to the active pharmaceutical ingredient lies in a range of 1:1 to 15:1, and wherein the coating pan rotates at 10 revolutions per minute or faster.

10. The process for the preparation of a pharmaceutical single unit dosage form according to claim 8 or 9, wherein the core comprises an angiotensin II antagonist as a active pharmaceutical ingredient, preferably selected from the group consisting of sartans (preferably telmisartan, losartan, eprosartan, irbesartan, valsartan and candesartan), cholesterol absorption inhibitors (preferably ezetimibe); and statins (preferably atorvastatin, simvastatin, rosuvastatin and lovastatin); most preferably being telmisartan.

11. The process for the preparation of a pharmaceutical single unit dosage form according to any one of claims 8 to 10, wherein the film coating comprises a diuretic as a active pharmaceutical ingredient, preferably selected from the group consisting of hydrochlorothiazide, chlorothiazide and trichlorothiazide and their salts, more preferably hydrochlorothiazide; or calcium channel blockers, preferably amlodipine, lercanidipine, nimodipine, nifedipine, verapamil and diltiazem; in particular wherein said second active pharmaceutical ingredient is hydrochlorothiazide or its salt.

12. A set of multiple pharmaceutical single unit dosage forms, wherein each dosage form unit of the set comprises a film coating comprising at least one active pharmaceutical ingredient and a co-polymer of polyvinyl alcohol with polyethylene glycol, wherein the weight ratio of the co-polymer to the active pharmaceutical ingredient in the film coating lies in a range of 1:1 to 15:1 and wherein the relative standard deviation of content variation of the active pharmaceutical ingredient in film coatings of said set is ≤ 7 %.

13. The set of multiple pharmaceutical single unit dosage forms according to claim 12, wherein each dosage form unit of the set comprises a film coating obtained by providing a core of the single unit dosage form in a coating pan, optionally providing one or more subcoating layer(s) on the core, and subjecting the core, optionally provided with one or more subcoating layer(s), to film coating using a composition comprising an aqueous coating vehicle, a co-polymer of polyvinyl alcohol with polyethylene glycol and at least one active pharmaceutical ingredient dispersed in said aqueous coating vehicle, wherein the coating pan rotates at 10 revolutions per minute or faster.

14. Use of a composition comprising a co-polymer of polyvinyl alcohol with polyethylene glycol and an aqueous coating vehicle, which comprises at least one active pharmaceutical ingredient, wherein said at least one active pharmaceutical ingredient is defined by a low water solubility of about 10mg/m or lower as measured in water at 20°C at about pH 7, or which is defined by presenting a dispersed state when placed in water at 20°C at about pH 7, and the weight ratio of said co-polymer to the active pharmaceutical ingredient in the vehicle lies in a range of 1:1 to 15:1, in a film coating said active pharmaceutical ingredient onto a pharmaceutical single unit dosage form.

15. Use according to claim 14, wherein said at least one active pharmaceutical ingredient is hydrochlorothiazide.
